# EUROPEAN PATENT APPLICATION

(11) **EP 4 717 255 A1**
(43) Date of publication of application: **01.04.2026**
(21) Application number: 24306590.1
(22) Date of filing: 27.09.2024
(51) Int. Cl.: A61K 8/9717, A61Q 19/08

(54) **GRATELOUPIA TURUTURU EXTRACT AND COSMETIC USE THEREOF**

(71) Applicant: Laboratoires Gilbert, 14200 Herouville Saint Clair (FR)
(72) Inventor: CHOPIN, Sylvie, 14830 LANGRUNE-SUR-MER (FR); GARDERES, Johan, Jean-Claude, André, 14112 BIEVILLE-BEUVILLE (FR); HENNEQUART, Franck, Eric, Jean-Pierre, 14200 HEROUVILLE-SAINT-CLAIR (FR); BREBION, Jeremy, 14490 BALLEROY SUR DROME (FR); BENOIT, Maud, 22620 PLOUBAZLANEC (FR)
(74) Representative: Lavoix

(57) **Abstract**

The present invention relates to a *Grateloupia turuturu* red algae extract and a cosmetic composition comprising thereof. The present invention also relates to a process for preparing a *Grateloupia turuturu* red algae extract by aqueous hot extraction. The present invention also relates to the use of said *Grateloupia turuturu* red algae extract as a cosmetic active ingredient, in particular as a slow-ageing agent.

## Description

### Field of the invention

The present invention relates to the field of active ingredients for cosmetic applications.

### Technical background

*Grateloupia turuturu,* also known as Devil's Tongue Weed or Asian red seaweed, is a red algae originating from Japan, where it is used as food. However, *Grateloupia turuturu* is most known as an invasive species on the Atlantic coast. This species is now growing naturally along the shores from the north of Ireland to the west coast of Portugal with however limited biomass available as a wild stock. This species is also cultivated by several companies in Europe. Red seaweed polysaccharides (in particular galactan) have been studied for anti-bacterial and anti-allergic properties.

Consumers are looking for natural products in all areas, particularly in food and cosmetics. In cosmetics, plant derived ingredients are widely used. Plants may indeed constitute a natural source of many useful active substances. However, there are many difficulties and pitfalls, in order to successfully select a plant containing useful active substances and determining in which form said active substances can be provided to exert their cosmetic effect, while being able to be industrially produced and formulated.

As regard to slow ageing, aged skin is particularly characterized by wrinkles, hyperpigmentation, a reduced number and activity of the fibroblasts, a loss of dermal adipocytes and an overall thickness decrease, by comparison to normal skin. The physiology of aged skin particularly results from an increase of pro-inflammatory molecules, free radicals and extracellular proteases, leading to an inflammation, oxidative stress and disorganization of the extracellular matrix, respectively. Useful slow-ageing agents thus particularly include ingredients able to inhibit or reduce expression of extracellular proteases and/or reduce oxidative stress.

There is still a need for natural active ingredients for cosmetic applications, in particular for slow ageing applications.

### Description of the invention

The Inventors have unexpectedly found that *Grateloupia turuturu* extracts have interesting cosmetic properties, in particular slow-ageing properties.

The *Grateloupia turuturu* extracts may advantageously be obtained by a process which is easy to implement and is based on an aqueous hot extraction, in particular under stirring, followed by a solid / liquid separation and a frontal filtering step.

Depending on the desired biological activity of the *Grateloupia turuturu* extract, the method may further comprise one or two tangential ultrafiltering and diafiltering steps.

As shown in the Examples, the *Grateloupia turuturu* extracts according to the invention advantageously inhibit at least one extracellular protease, in particular collagenase and/or elastase, and/or at least one glycosidase, in particular hyaluronidase. Some of these extracts also reduce expression of extracellular proteases, inhibit oxidative stress and/or induce cell proliferation of senescent fibroblasts. The *Grateloupia turuturu* extracts according to the invention are thus slow-ageing agents, which can be used as active ingredient in cosmetic compositions, in particular for slow ageing applications.

A first object of the invention is a red algae extract, wherein said red algae is *Grateloupia turuturu.*

The red algae extract as defined above is preferably obtained by a method comprising an aqueous hot extraction, a solid / liquid separation and a filtration.

The red algae extract as defined above may have the following properties:
- inhibition of at least one enzyme selected from the group consisting of collagenase, elastase and hyaluronidase,
- optionally, reduction of the expression of at least one extracellular protease, said extracellular protease being selected from the group consisting of ADAMT5, MMP2 and MMP3,
- optionally, induction of cell proliferation of senescent fibroblasts, and
- optionally, decrease of oxidative stress.

Another object of the invention is a cosmetic composition comprising the red algae extract as defined above.

Another object of the invention is a process for preparing a *Grateloupia turuturu* red algae extract, said process comprising:
a) providing *Grateloupia turuturu* red algae,
b) subjecting said red algae to an aqueous hot extraction, thereby obtaining a mixture,
c) subjecting said mixture to a solid / liquid separation, thereby obtaining a liquid fraction,
d) filtering said liquid fraction, thereby obtaining a clarified liquid fraction,
e) optionally, tangential ultrafiltering and diafiltering said clarified liquid fraction, thereby obtaining a first retentate and a first permeate, and
f) optionally, tangential ultrafiltering and diafiltering said first permeate, thereby obtaining a second retentate and a second permeate.

The algae provided in step a) may be (i) fresh algae, (ii) fresh, freezed and defrosted algae or (iii) dried algae.

The aqueous hot extraction in step b) may comprise macerating the red algae in water at a temperature comprised from 50°C to 80°C.

The solid / liquid separation in step c) may be a centrifugation or a decantation.

Tangential ultrafiltering and diafiltering in step e) may be carried out with a filter having a pore size of 150 kDa and/or tangential ultrafiltering and diafiltering in step f) is carried out with a filter having a pore size of 3 Da.

Another object of the invention is a process for preparing a cosmetic composition, wherein said process comprises mixing the red algae extract as defined above or obtained by the process as defined above with at least one other cosmetic ingredient.

Another object of the invention is a cosmetic method comprising applying topically a cosmetic composition as defined above or obtained by the process as defined above, in particular for slowing ageing of the skin.

Another object of the invention is the use of a red algae extract as defined above or obtained by the process as defined above, as a cosmetic active ingredient, in particular as a slow-ageing agent.

### Grateloupia turuturu

The red alga used as a starting material for preparing the red algae extract according to the invention is *Grateloupia turuturu.*

*Grateloupia turuturu* is a red seaweed species containing sulfated polysaccharides, especially galactan compounds, proteins and minerals.

*Grateloupia turuturu* is also known as *Halymenia sinensis.*

The expressions *"Grateloupia turuturu" and "Grateloupia turuturu red algae"* are herein synonymous.

The terms "algae" and "seaweed" are herein synonymous.

*Grateloupia turuturu* may be harvested from its natural environment, such as from the sea, ocean or coast, or from a cultivation area.

*Grateloupia turuturu* may for example be cultivated algae, for example cultured in a cultivation area, such as a pond, a marine lagoon or a land-based cultivation.

*Grateloupia turuturu* may be harvested from its natural environment or cultivation area by any suitable method, such as collecting free-floating algae, collecting the algal biomass from nets or ropes or harvested by hand on the beach.

Cultivated *Grateloupia turuturu* algae may be harvested, in particular from the cultivation area, by any suitable method, such as suction followed by filtration and draining, or by hand, for example with a net.

The harvested algae are also referred to as biomass.

*Grateloupia turuturu* may be used in the form of fresh algae or dry algae. When using fresh algae, they are preferably freezed and defrosted.

By "fresh algae", it is herein means algae as harvested, in particular without further processing such as drying or freezing.

Fresh algae typically have a water content greater than or equal to 75%, preferably greater than 80%, such as greater than 85%. The percentage is a mass percentage.

By "fresh, freezed and defrosted algae", it is herein meant fresh algae as defined above submitted to a step of freezing followed by a step of defrosting.

By "dry algae", it is herein meant algae obtained by drying fresh algae.

Dry algae may be obtained by any drying method well known by the skilled person, such as freeze-drying, oven drying, drum drying or tunnel drying.

Dry algae are preferably obtained by drying at up to 40°C. The drying time is preferably as short as possible. Drying is followed by grinding.

Dry algae typically have a water content of less than or equal to 15%, preferably less than 10% or less than 5%. The percentage is a mass percentage.

### Grateloupia turuturu extract

The present invention particularly relates to a red algae extract, which is a *Grateloupia turuturu* extract.

The *Grateloupia turuturu* extract is obtained from a *Grateloupia turuturu* red algae as defined above.

The expressions *"Grateloupia turuturu* extract" and *"Grateloupia turuturu* red algae extract" are herein synonymous.

The *Grateloupia turuturu* extract is preferably obtained by an aqueous hot extraction, in particular by macerating *Grateloupia turuturu* red algae in hot water under stirring, preferably at a temperature comprised from 50°C to 80°C, more preferably from 60°C to 75°C, still more preferably from 65°C to 70°C, for example at 70°C. Aqueous hot extraction is particularly as defined below in the section "Process for preparing a *Grateloupia turuturu* extract", step b).

When using fresh algae, they are preferably cut into small pieces, preferably smaller than 10 cm in length, more preferably smaller than 5 cm in length, such as pieces of from 0,5 cm to 1 cm, before being macerated in hot water under stirring as defined above. Still preferably, when using fresh algae, they are preferably freezed and defreezed, then cut in small pieces as defined above, then macerated in hot water under stirring as defined above.

Interestingly, different active extracts are obtained, depending on whether the starting material is fresh or dry *Grateloupia turuturu* red algae and on whether additional steps of ultrafiltration and diafiltration are carried out.

The *Grateloupia turuturu* extract may be obtained by a process as defined below in the section "Process for preparing a *Grateloupia turuturu* extract".

In one embodiment, the *Grateloupia turuturu* extract is the liquid fraction obtained by solid / liquid separation of a mixture (such as centrifugation), wherein said mixture is obtained by aqueous hot extraction of *Grateloupia turuturu* red algae. Said liquid fraction is then preferably clarified by filtration, in particular frontal filtration under between 300 µm and 800 µm sieve, such as under 400 µm sieve, to remove solid particles. This extract, also herein referred to as EX1 extract, is for example obtained in step d) of the process defined below for preparing a *Grateloupia turuturu* extract.

The EX1 extract obtained from fresh *Grateloupia turuturu* red algae for example comprises:
- between 25 and 35% of ashes,
- less than 10% of proteins,
- between 20% and 30% of neutral sugars (including between 15% and 25% of galactose and between 2% and 6% of glucose),
- less than 3% of uronic acids and
- less than 4% of floridoside.

The expression "neutral sugar", it is herein meant galactose, xylose, mannose, glucose or 6-O-methyl galactose.

The EX1 extract obtained from fresh *Grateloupia turuturu* red algae inhibits moderately collagenase and slightly elastase, decreases extracellular proteases and oxidative stress and induces cell proliferation of senescent fibroblasts.

The EX1 extract obtained from dried *Grateloupia turuturu* red algae for example comprises:
- between 30 and 45% of ashes,
- between 5% and 15% of proteins,
- between 20% and 30% of neutral sugars (including between 15% and 25% of galactose and between 4% and 10% of glucose),
- less than 3% of uronic acids and
- less than 3% of floridoside.

The EX1 extract obtained from dry *Grateloupia turuturu* red algae inhibits moderately collagenase and slightly elastase, decreases expression extracellular proteases gene and oxidative stress.

In another advantageous embodiment, the *Grateloupia turuturu* red algae extract is the retentate obtained by tangential ultrafiltering and diafiltering, in particular using a ceramic membrane, i.e. by tangential ultrafiltering and diafiltering a liquid fraction, wherein said liquid fraction is obtained by solid / liquid separation (such as centrifugation) of a mixture, wherein said mixture is obtained by aqueous hot extraction of *Grateloupia turuturu* red algae. This *Grateloupia turuturu* extract, also referred to as RE1 extract, is for example obtained in step e) of the process for preparing a *Grateloupia turuturu* extract as defined below.

The RE1 extract does not comprise compounds of a size lower than or equal to 150 kDa.

The compounds of the RE1 extract thus have a size greater than 150 kDa.

The RE1 extract obtained from fresh *Grateloupia turuturu* red algae for example comprises:
- between 10% and 15% of ashes,
- between 10 and 18% of proteins,
- between 35% and 45% of neutral sugars (including between 30% and 40% of galactose and between 3% and 8% of glucose),
- between 1% and 3% of uronic acids and
- less than 3% of floridoside.

The RE1 extract obtained from fresh *Grateloupia turuturu* red algae inhibits slightly collagenase, highly hyaluronidase and slightly elastase, decreases the expression of extracellular proteases, decreases oxidative stress and induces cell proliferation of senescent fibroblasts.

The RE1 extract obtained from dried *Grateloupia turuturu* red algae for example comprises:
- between 10% and 20% of ashes,
- between 10% and 20% of proteins,
- between 35% and 50% of neutral sugars (including between 35% and 45% of galactose and between 1% and 8% of glucose),
- between 1% and 3% of uronic acids and
- less than 3% of floridoside.

The RE1 extract obtained from dry *Grateloupia turuturu* red algae inhibits moderately collagenase and strongly hyaluronidase, decreases expression of extracellular proteases, decreases oxidative stress and induces cell proliferation.

In one other advantageous embodiment, the *Grateloupia turuturu* extract is the permeate obtained by tangential ultrafiltering and diafiltering (in particular using a ceramic membrane), i.e. by tangential ultrafiltering and diafiltering a liquid fraction, wherein said liquid fraction is obtained by solid / liquid separation (such as centrifugation) of a mixture, wherein said mixture is obtained by aqueous hot extraction of *Grateloupia turuturu* red algae. This extract, also referred to as PE1 extract, is for example obtained in step e) of the process for preparing a *Grateloupia turuturu* extract as defined below.

The PE1 extract does not comprise compounds of a size greater than 150 kDa.

The compounds of the PE1 extract indeed have a size lower than or equal to 150 kDa.

The PE1 extract obtained from fresh *Grateloupia turuturu* red algae for example comprises:
- between 60 and 70% of ashes,
- less than 3% of proteins,
- less than 10% of neutral sugars (including between 2% and 8% of galactose and between 2% and 8% of glucose),
- less than 3% of uronic acids and
- between 4% and 10% of floridoside.

The PE1 extract obtained from fresh *Grateloupia turuturu* red algae inhibits slightly collagenase and slightly elastase.

The PE1 extract obtained from dried *Grateloupia turuturu* red algae for example comprises:
- between 60 and 70% of ashes,
- less than 3% of proteins,
- between 4% and 10% of neutral sugars (including between 1% and 5% of galactose and between 3% and 9% of glucose),
- less than 3% of uronic acids and
- less than 10% of floridoside.

The PE1 extract obtained from dry *Grateloupia turuturu* red algae inhibits moderately collagenase and slightly elastase.

In one other advantageous embodiment, the *Grateloupia turuturu* extract is the retentate obtained by tangential ultrafiltering and diafiltering, in particular using a ceramic membrane, i.e. by tangential filtering and diafiltering a first permeate, wherein said first permeate is obtained by tangential ultrafiltering and diafiltering, in particular using a ceramic membrane, a liquid fraction, wherein said liquid fraction is obtained by solid/liquid separation (such as centrifugation) of a mixture, wherein said mixture is obtained by aqueous hot extraction of *Grateloupia turuturu* red algae. This extract, also referred to as RE2 extract, is for example obtained in step f) of the process for preparing a *Grateloupia turuturu* extract as defined below.

The RE2 extract does not comprise compounds of a size lower than or equal to 3 Da, nor compounds of a size greater than 150 kDa.

The compounds of the RE2 extract have a size greater than 3 kDa and lower than or equal to 150 kDa.

The RE2 extract obtained from fresh *Grateloupia turuturu* red algae for example comprises:
- between 15 and 25% of ashes,
- less than 3% of proteins,
- between 5% and 15% of neutral sugars (including between 1% and 4% of galactose and between 4% and 9% of glucose),
- between 1% and 3% of uronic acids and
- less than 1% of floridoside.

The RE2 extract obtained from fresh *Grateloupia turuturu* red algae inhibits moderately collagenase and slightly elastase.

The RE2 extract obtained from dried *Grateloupia turuturu* red algae for example comprises:
- between 15% and 25% of ashes,
- less than 4% of proteins,
- between 5% and 15% of neutral sugars (including between 1% and 5% of galactose and between 1% and 5% of glucose),
- less than 1% of uronic acids, and
- less than 1% of floridoside.

The RE2 extract obtained from dry *Grateloupia turuturu* red algae inhibits strongly collagenase and slightly elastase.

In one other advantageous embodiment, the *Grateloupia turuturu* red algae extract is the permeate obtained by tangential ultrafiltering and diafiltering the PE1 extract, in particular using a ceramic membrane, i.e. by tangential ultrafiltering and diafiltering a first permeate, wherein said first permeate is obtained by tangential filtering and diafiltering a liquid fraction, wherein said liquid fraction is obtained by solid/liquid separation (such as centrifugation) of a mixture, wherein said mixture is obtained by aqueous hot extraction of *Grateloupia turuturu* red algae. This extract, also referred to as PE2 extract, is for example obtained in step f) of the process for preparing a *Grateloupia turuturu* extract as defined below.

The PE2 extract does not comprise compounds of a size greater than 3 Da.

The compounds of the PE2 extract have a size lower than or equal to 3 Da.

The PE2 extract obtained from fresh *Grateloupia turuturu* red algae for example comprises:
- between 70% and 80% of ashes,
- less than 3% of proteins,
- less than 10% of neutral sugars (including between 4% and 8% of galactose),
- less than 3% of uronic acids and
- less than 10% of floridoside.

The PE2 extract obtained from fresh *Grateloupia turuturu* red algae inhibits slightly collagenase and slightly elastase and reduces oxidative stress.

The PE2 extract obtained from dried *Grateloupia turuturu* red algae for example comprises:
- between 75% and 85% of ashes,
- less than 3% of proteins,
- less than 5% of neutral sugars,
- less than 3% of uronic acids and
- less than 5% of floridoside.

The PE2 extract obtained from dry *Grateloupia turuturu* red algae inhibits slightly elastase.

The above percentages of ashes, proteins, neutral sugars, uronic acids and floridoside are expressed in weight of dry matter.

All the extracts defined above, in particular EX1, RE1, PE1, RE2 and PE2 extracts, comprise no or non-significant amounts of phenol compounds and digeneasides. By "non-significant amount", it is herein meant less than 0,01% in weight of dry matter.

All the *Grateloupia turuturu* extracts defined above, in particular EX1, RE1, PE1, RE2 and PE2 extracts, have interesting biological properties. The *Grateloupia turuturu* extracts as defined above indeed inhibit at least one extracellular protease, such as collagenase, and elastase and/or at least one glycosidase, such as hyaluronidase. Some of these extracts additionally reduces expression of extracellular protease(s), such as ADAMT5, MMP2 and/or MMP3, reduce oxidative stress and/or induce cell proliferation of senescent fibroblasts.

Inhibition of collagenase, hyaluronidase or elastase may be assessed by any assay well known by the skilled person, such as assessing the inhibition activity of a product to be assessed on the enzyme activity, for example *in tubo.* A positive control (using an inhibiting molecule) and a negative control (without molecules) is used to validate the experiment.

Reduced expression of extracellular protease(s), such as ADAMT5 (*disintegrin and metalloproteinase with thrombospondin motifs 5*), MMP2 (*matrix metallopeptidase 2*) and/or MMP3 (*matrix metallopeptidase 3*), may be assessed by any method well known by the skilled person. For example, their respective mRNA expression is measured in senescent fibroblasts, for example using real-time polymerase chain reaction (RT-PCR). The results obtained in the presence of the product to be tested are compared to those obtained in its absence.

Reduced oxidative stress may be assessed by any method well known by the skilled person, such as DPPH (2,2-diphenyl-1-picrylhydrazyle) reduction test or Fenton reactions with divalent cations. DPPH reduction test measures the antioxidant activity of compounds capable of transferring hydrogen atoms. Compound (DPPH°+) is a stable, purple-colored radical cation that shows a maximum absorbance at 517 nm. If the compound to be tested is an antioxidant compound able to transfer an electron to DMPD°+, the solution is discolored. This reaction is rapid and proportional to the antioxidant capacity of the product to be tested. In the Fenton reaction, the chelation reaction of Fe²⁺ and Cu²⁺ ions is measured colorimetrically. These ions have their own absorbances. Chelation of these ions by a product to be tested shifts the absorbance of the complexed ion. The shift rate of the absorbance peak is proportional to the percentage of ion complexation.

Induced cell proliferation of senescent fibroblasts may be assessed by any method well known by the skilled person, such as by assessing cellular density by microscopy.

The *Grateloupia turuturu* extract as defined above preferably has the following properties:
- inhibition of at least one extracellular protease, said extracellular protease being preferably collagenase or elastase and/or of at least one glycosidase, said glycosidase being hyaluronidase,
- optionally, reduction of the expression of at least one extracellular protease, said extracellular protease being preferably selected from the group consisting of ADAMT5, MMP2 and MMP3,
- optionally, induction of cell proliferation, in particular of cell proliferation of senescent fibroblasts, and
- optionally, decrease of oxidative stress.

The *Grateloupia turuturu* extracts defined above, in particular EX1, RE1, PE1, RE2 and PE2 extracts, are thus cosmetic active ingredients useful for cosmetic applications, preferably slow-ageing applications.

The *Grateloupia turuturu* extract is preferably selected from the group consisting of EX1 extract, RE1 extract and RE2 extract, more preferably from the group consisting of EX1 extract obtained from fresh or dried algae, RE1 extract obtained from fresh or dried algae and RE2 extract obtained from dried algae.

In a still preferred embodiment, the *Grateloupia turuturu* extract is RE1 extract obtained from fresh algae.

### Cosmetic composition

The present invention also relates to a cosmetic composition comprising a *Grateloupia turuturu* extract as defined above.

The *Grateloupia turuturu* extract is thus used in the cosmetic composition as an active ingredient, more preferably as a slow-ageing agent.

By "slow-ageing agent", it is herein meant an ingredient having at least one of the following properties, preferably at least two, more preferably at least three of the following properties:
- inhibiting at least one extracellular protease, said extracellular protease being preferably collagenase or elastase and/or at least one glycosidase, said glycosidase being preferably hyaluronidase,
- reducing the expression of at least one extracellular protease, said extracellular protease being preferably selected from the group consisting of ADAMT5, MMP2 and MMP3,
- inducing cell proliferation, in particular cell proliferation of senescent fibroblasts, and/or
- decreasing oxidative stress.

Inhibiting elastase also increases the amount of elastin, which allows reorganization of the matrix.

The slowing-ageing properties of the *Grateloupia turuturu* extracts according to the invention are as particularly as defined above.

The cosmetic composition as defined above preferably comprise at least 0,1% of a *Grateloupia turuturu* extract as defined above, preferably at least 1%, at least 2%, at least 5%, at least 8% or at least 10% of a *Grateloupia turuturu* extract as defined above.

In one alternative embodiment, the cosmetic composition as defined above may comprise at least 15%, at least 20%, at least 25%, at least 30%, at least 35%, at least 40%, at least 45%, more preferably still at least 50% of a *Grateloupia turuturu* extract as defined above.

In yet another alternative embodiment, the cosmetic composition as defined above may comprise at least 55%, at least 60%, at least 65%, at least 70%, at least 75%, at least 80%, at least 85%, more preferably still at least 90% of a *Grateloupia turuturu* extract as defined above.

A preferred cosmetic composition for example comprises from 1% to 99%, preferably from 1,5% to 99%, preferably from 2% to 99%, more preferably from 2% to 90%, more preferably from 2% to 80% , more preferably from 2% to 70%, more preferably from 2% to 60%, more preferably from 2% to 50% of a *Grateloupia turuturu* extract as defined above.

Another preferred cosmetic composition for example comprises from 0,1% to 20%, such as from 0,1% to 15% or from 0,1% to 10% of a *Grateloupia turuturu* extract as defined above.

Another preferred cosmetic composition for example comprises from 2% to 10%, from 10% to 20%, from 20% to 30%, from 30% to 40% or from 40% to 50% of a *Grateloupia turuturu* extract as defined above.

Another preferred cosmetic composition for example consists of a *Grateloupia turuturu* extract as defined above.

The above percentages are expressed by mass over the total mass of the cosmetic composition.

The cosmetic composition as defined above may comprise at least one other cosmetic ingredient.

Said other cosmetic ingredient may be an active ingredient, an excipient and/or an additive.

Said other active ingredient may for example be selected from the group consisting of a moisturizing agent, such as glycerin or hyaluronic acid.

Said other active ingredient may for example be a skin substantive agent. By "skin substantive agent" as active ingredient, it is herein meant an agent having at least one activity complementary to those of the *Grateloupia Turuturu* extract as defined above. Said other active ingredient may for example be selected from the group consisting of a moisturizing agent, a soothing agent and skin-protecting agent.

The excipient may for example be selected from the group consisting of a solvent, an emulsifier, a stabilizing agent (e.g. an emulsion stabilizer), a gelling agent, a surfactant and an emollient and combinations thereof.

The additive may, for example, be selected from the group consisting of an antioxidant agent, a chelating agent, a masking agent, a preservative (for example an antibacterial and/or antifungal agent), a colorant, a perfume and combinations thereof.

The solvent may for example be water and/or propanediol.

The cosmetic composition as defined above preferably comprises:
- a *Grateloupia turuturu* extract as defined above,
- at least one excipient, preferably at least two excipients, more preferably at least three excipients, and
- optionally, at least one other active ingredient.

The cosmetic composition as defined above may for example comprise:
- a *Grateloupia turuturu* extract as defined above,
- at least one vegetable oil and/or a wax,
- at least one antioxidant, such as vitamin E and/or a polyphenol,
- at least one moisturizing agent, and
- optionally, at least one additive, such as a preservative, colorant or perfume,
said cosmetic composition having preferably a pH that respects the skin where it is applied, for example an acidic or neutral pH, for example comprised from 4,5 to 7,5.

The cosmetic composition as defined above is preferably suitable for a topical application, i.e. an application on the skin.

The cosmetic composition as defined above can be a lotion, an oil (preferably lipid-replenishing), a balm, a cream, a gel, a sunscreen or a mask.

The cosmetic composition as defined above may be in liquid, semi-liquid, solid, powder, micellar, gel or cream form.

The cosmetic composition as defined above preferably has a pH compatible with a topical use on the skin.

In a particular embodiment, the cosmetic composition as defined above has an acidic pH, preferably comprised from pH 4.5 to 5.

### Process for preparing a cosmetic composition

The present invention also relates to a process for preparing a cosmetic composition as defined above.

The process for preparing a cosmetic composition as defined above preferably comprises a step of mixing a *Grateloupia turuturu* extract as defined above with at least one other cosmetic ingredient.

The *Grateloupia turuturu* extract, the cosmetic composition and the other cosmetic ingredient are particularly as defined above.

### Process for preparing a Grateloupia turuturu extract

The present invention particularly relates to a process for preparing a *Grateloupia turuturu* extract as defined above.

The process for preparing a *Grateloupia turuturu* red algae extract preferably comprises:
a) providing *Grateloupia turuturu* red algae,
b) subjecting said red algae to an aqueous hot extraction, thereby obtaining a mixture,
c) subjecting said mixture to a solid / liquid separation, thereby obtaining a liquid fraction,
d) filtering said liquid fraction, thereby obtaining a clarified liquid fraction,
e) optionally, tangential ultrafiltering and diafiltering said clarified liquid fraction, thereby obtaining a first retentate and a first permeate, and
f) optionally, tangential ultrafiltering and diafiltering said first permeate, thereby obtaining a second retentate and a second permeate.

### Step a)

Step a) comprises providing *Grateloupia turuturu* red algae.

Said *Grateloupia turuturu* red algae are as defined above.

Said *Grateloupia turuturu* red algae may be (i) fresh algae, (ii) fresh, freezed and defrosted algae or (iii) dried algae.

Fresh *Grateloupia turuturu* red algae are particularly as defined above.

Fresh *Grateloupia turuturu* red algae may be obtained by harvesting, as defined above.

Fresh *Grateloupia turuturu* red algae may first be washed with seawater to remove particles.

When using fresh algae, *Grateloupia turuturu* red algae are preferably cut in small pieces, preferably smaller than 10 cm in length, more preferably small than 5 cm in length, such as for example from 0,5 to 1 cm in length.

When using fresh algae, the *Grateloupia turuturu* red algae are preferably freezed and defrosted, in particular before being cut in small pieces as defined above. Freezing and defrosting allow bursting the cells and freezing advantageously allows storing the algae before use.

When using fresh algae, step a) may thus comprise a step of freezing and defreezing fresh *Grateloupia turuturu* red algae and/or a step of cutting *Grateloupia turuturu* red algae in small pieces, preferably smaller than 10 cm in length, more preferably smaller than 5 cm in length, such as for example from 0,5 to 1 cm in length.

Dried *Grateloupia turuturu* red algae are particularly as defined above.

Dried *Grateloupia turuturu* red algae may be obtained as defined above, for example by freeze-drying, oven drying, drum drying or tunnel drying, preferably followed by grinding.

When using dry algae, step a) may comprise drying *Grateloupia turuturu* red algae, followed preferably by grinding.

### Step b

Step b) comprises subjecting said red algae to an aqueous hot extraction, thereby obtaining a mixture.

Aqueous extraction as defined above is for example performed a temperature comprised from 50°C to 80°C, more preferably from 60°C to 75°C, still more preferably from 65 to 70°C, for example at 70°C.

Aqueous extraction as defined above is preferably carried out at a slightly acidic to neutral pH, more preferably at a pH lower or equal to 7, for example at a pH comprised from 5 to 7.

pH is preferably not adjusted during step b).

Aqueous hot extraction as defined above is preferably carried out during at least two hours, preferably at least four hours or at least six hours, and/or during at most ten hours. Aqueous hot extraction as defined above is for example carried out between four hours and six hours.

Aqueous extraction as defined above preferably comprises macerating the red algae in hot water, preferably under stirring.

Aqueous extraction as defined above preferably comprises macerating the red algae in hot water in the following conditions:
- under stirring,
- at a temperature comprised from 50°C to 80°C, more preferably from 60°C to 75°C, still more preferably from 65 to 70°C, for example at 70°C,
- at a slightly acidic to neutral pH, more preferably at a pH lower or equal to 7, for example at a pH comprised from 5 to 7, and/or
- during at least two hours, preferably at least four hours or at least six hours, and/or during at most ten hours.

Typically, the weight ratio fresh algae / water is around 1:4. For example, 4 kg of water for 1 kg of fresh algae is used in step b).

Typically, the weight ratio dry algae / water is around 1:25. For example, 25 kg of water for 1 kg of dry algae is used in step b).

In a preferred embodiment, aqueous hot extraction as defined above comprises macerating the red algae in water at 70°C, at a pH comprised from 5 to 7, for four to six hours and under stirring.

### Step c)

Step c) comprises subjecting the mixture obtained in step b) to a solid / liquid separation, thereby obtaining a liquid fraction.

The solid / liquid separation may be obtained by any method well known by the skilled person, such as centrifugation or decantation.

The solid / liquid separation is preferably a centrifugation.

The solid / liquid separation allows obtaining a liquid fraction and a solid fraction and the liquid fraction is collected.

Step c) is preferably carried out at room temperature, in particular between 18°C and 22°C.

The liquid fraction obtained in step c) comprises cosmetic active ingredient(s).

### Step d)

Step d) comprises filtering the liquid fraction obtained in step c), thereby obtaining a clarified liquid fraction.

The filtering step is preferably a frontal filtration (also called dead-end filtration). In frontal filtering, the liquid fraction flows vertically through the membrane.

This filtering step allows removing remaining solid residues.

Filtering is preferably performed on a filter having a pore size of between 300 and 800 µm, such as 400 µm.

Step d) is preferably carried out at a temperature greater than or equal to 40°C. A temperature greater than or equal to 40°C particularly allows avoiding problems resulting from the viscosity of the liquid fraction during the filtration step.

Step d) is preferably carried out between 40°C and 60°C.

The liquid fraction is collected and corresponds to the clarified liquid fraction.

The clarified liquid fraction comprises cosmetic active ingredient(s).

The clarified liquid fraction obtained in step d), which is also referred to as EX1 extract, corresponds to a *Grateloupia turuturu* red algae extract according to the invention.

The EX1 extract is particularly as defined above.

### Step e)

Optional step e) comprises tangential ultrafiltering and diafiltering said clarified liquid fraction, thereby obtaining a first retentate and a first permeate.

This ultrafiltering and diafiltering step allows separating particles having a size greater to the pore size of the filter and those have a size equal to or lower than the pore size of the filter.

Ultrafiltering and diafiltering is preferably performed with a filter having a pore size of 150 kDa. Said filter may be a membrane, such as a ceramic membrane.

Basically, the process fluid (initially the clarified liquid fraction) flows tangentially to the filter. The retentate is recirculated into the process fluid and a volume of water is continuously added into the process fluid, said volume being equal to the volume of permeate which is removed.

The ultrafiltering and diafiltering step is ended when the conductivity of the permeate reaches the conductivity of the water initially used to carry out the diafiltration.

The ultrafiltering and diafiltering step is for example ended when the conductivity of the permeate is between 250 µS/cm and 350 µS/cm, such as around 300 µS/cm.

Step e) is carried out at a temperature lower than or equal to 40°C.

Step e) is preferably carried out at a temperature between 25°C and 40°C.

The ultrafiltering and diafiltering step allows obtaining a first permeate and a first retentate.

The first retentate comprises cosmetic active ingredient(s).

The first retentate obtained in step e), which is also referred to as RE1 extract, corresponds to a *Grateloupia turuturu* red algae extract according to the invention.

The first permeate also comprises cosmetic active ingredient(s).

The first permeate obtained in step e), which is also referred to as PE1 extract, corresponds to a *Grateloupia turuturu* red algae extract according to the invention.

### Step f)

Optional step f) comprises tangential ultrafiltering and diafiltering said first permeate, thereby obtaining a second retentate and a second permeate.

When step f) is carried out, the method comprises step e).

This second ultrafiltering and diafiltering step allows separating particles having a size greater to the pore size of the filter and those have a size equal to or lower than the pore size of the filter.

Ultrafiltering and diafiltering is preferably performed with a filter having a pore size of 3 Da. Said filter may be a membrane, such as a ceramic membrane.

Basically, the process fluid (initially the first permeate) flows tangentially to the filter. The retentate is recirculated into the process fluid and a volume of water is continuously added into the process fluid, said volume being equal to the volume of permeate which is removed.

The ultrafiltering and diafiltering step is ended when the conductivity of the permeate reaches the conductivity of the water initially used to carry out the diafiltration.

The ultrafiltering and diafiltering step is for example ended when the conductivity of the permeate is between 250 µS/cm and 350 µS/cm, such as around 300 µS/cm.

Step e) is preferably carried out at room temperature, in particular between 18°C and 22°C.

The ultrafiltering and diafiltering step allows obtaining a second permeate and a second retentate.

The second retentate comprises cosmetic active ingredient(s).

The second retentate obtained in step f), which is also referred to as RE2 extract, corresponds to a *Grateloupia turuturu* red algae extract according to the invention.

The second permeate also comprises cosmetic active ingredient(s).

The second permeate obtained in step f), which is also referred to as PE2 extract, corresponds to a *Grateloupia turuturu* red algae extract according to the invention.

### Use of a Grateloupia turuturu extract as a cosmetic active ingredient

The present invention also relates to the use of a *Grateloupia turuturu* extract as defined above as a cosmetic active ingredient, in particular in a cosmetic composition, preferably suitable for a topic use.

By the expression "cosmetic use", it is herein meant a non-therapeutic cosmetic use.

The *Grateloupia turuturu* extract is as defined above and may for example be obtained by the method for preparing a *Grateloupia turuturu* extract as defined above.

The cosmetic composition is particularly as defined above.

The present invention particularly relates to the use of a *Grateloupia turuturu* extract as defined above as a slow-ageing ingredient, in particular in a cosmetic composition as defined above, preferably suitable for a topic use.

### Cosmetic treatment method

The present invention also relates to a cosmetic treatment method, wherein said method comprises the topical application of a cosmetic composition as defined above.

By the expression "cosmetic treatment method", it is herein meant a non-therapeutic cosmetic treatment method.

By the expression "topical application", it is herein meant the application to the skin and skin appendages.

The skin encompasses the scalp.

Skin appendage includes hair and nails.

The cosmetic treatment method is preferably used in human being.

By the expression "human being", it is herein meant herein a human subject (also called an individual), for example a man or a woman, preferably adults and the elderly.

The human subject is preferably older than 20 years old, more preferably older than 25 years old, still more preferably older than 30, older than 40, older than 50, older than 60 or older than 70 years old. In a preferred embodiment, the human subject is at least 45 years old.

In a preferred embodiment, the human subject is not suffering from and/or is not likely to suffer from a dermatological disease.

As the *Grateloupia turuturu* extract has a slow-ageing effect, the present invention particularly relates to a cosmetic treatment method as defined above for slowing ageing of the skin.

The invention will be further illustrated in the following examples and figures.

### Brief description of the drawings

**Figure 1****:** Extraction process diagram starting from fresh or dry biomass of *Grateloupia turuturu.*
**Figure 2****:** Composition of the dried matter obtained from GT1 (A) and GT2 (B) extracts.
**Figure 3****:** Composition in saccharides of GT1 (A) and GT2 (B) extracts. The percentage is expressed on a dry matter basis. The sum of saccharides of each extract corresponds to the saccharide total of figure 2.
**Figure 4****:** Cell morphology of senescent fibroblast cells after 48h-incubation with or without extracts (objective x 4). A: control without extract, B: GT1-RE1, C: GT1-EX1, D: GT2-RE1, E: GT2-EX1, F: GT2-RE2.
**Figure 5****:** Orcein coloration of elastin fibers from senescent fibroblasts. A: control without extract, B: GT1-RE1, C: GT1-EX1, D: GT2-RE1, E: GT2-EX1, F: GT2-RE2. Scale bar: 1000 µm.

### EXAMPLES

### Material and Methods

### (i) Materials

Extractions were performed on the seaweed *Grateloupia turuturu.* The fresh biomass was collected along the Atlantic coast or cultivated in seawater tanks. The fresh biomass was freezed and defrosted or was dried before extraction, in order to study the extraction performed on both conditions. Extracts obtained from the fresh (freezed and defrosted) biomass were named with the prefix GT1-XXX and extracts obtained from the dried biomass were named GT2-XXX.

### (ii) Extraction

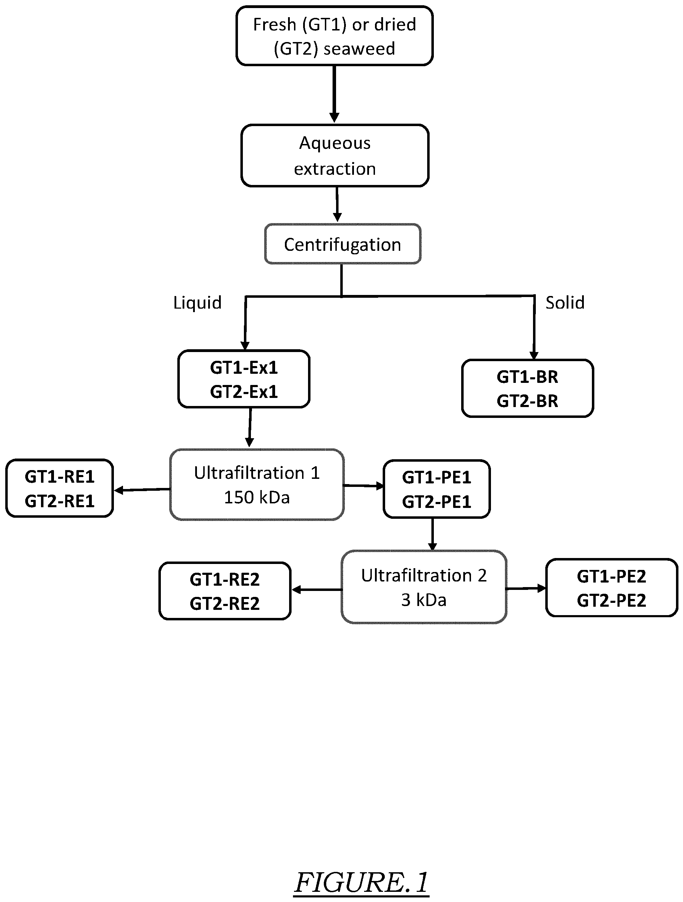

The extraction process is illustrated in Figure 1. The extraction process was performed both on fresh (freezed and defrosted) and dried biomasses. Selection of the candidate extracts was bio-guided using chemical and biological activity tests.

Fresh seaweed biomass was freezed and then defreezed, before to be cut in small pieces from 0.5 to 1 cm. The seaweed pieces were then macerated in water at 70 °C, pH 5-7, for 4 hours under stirring. The liquid fraction was collected after centrifugation step of the mix and filtrated at 400 µm. Solid residues were discarded. The clarified liquid fraction is name EX1. The EX1 fraction was then ultrafiltrated with a cutoff at 150 kDa followed by a diafiltration step. The retentate and the permeate were collected and named RE1 and PE1, respectively. The PE1 permeate was submitted to second ultrafiltration with a cutoff of 3 kDa followed by diafiltration step. The retentate and permeate were collected and named RE2 and PE2. A total of ten extracts were collected during the extraction process.

### (iii) Analytical characterization of the extracts

Extracts were freeze-dried and their chemical composition analyzed according to the size of the molecules present in the extract: saccharide composition (galactose, xylose, mannose, glucose and 6-O-methyl galactose), ashes, proteins, floridoside, digeneasides, phenolic compounds and uronic acids.

### (iv) Biological and chemical activities

All extracts were first studied with enzymatic and chemical *in tubo* tests. The anti-aging potential activities were assessed at 500 µg/mL of extracts with enzymatic inhibition tests performed on extracellular protein-degrading enzymes: collagenase, hyaluronidase and elastase. The five extracts, which provided the best results, were selected for further investigations (*see Table 1*). The cytotoxic and irritation potential effect of each extract also was assessed *in vitro* on Vero cells and on chorio-allantoic membranes (HET-CAM), respectively.

*In vitro* cellular tests were then performed on senescent fibroblasts (passage from p8 to p12), purchased at Sigma Aldrich and cultivated in DMEM + 10% Fetal Calf Serum (FCS) for 24h in order to induce cell senescence. The cell line displays a low proliferation rate, produces ROS and senescent-associated secretory phenotype (metalloproteases expression). The extracts were tested at a concentration of 5 % (v:v), corresponding to 625 µg/mL of total dry matter. In oxidative conditions, control and sample cell cultures were preincubated for 48 h with a sub lethal dose of H₂O₂ (0.475 M). After 48h of incubation without (control) or with the extracts, cells were washed and RNA was extracted. After reverse transcription, gene expression was assessed using real-time PCR and gene-dedicated primers. The expression of extracellular matrix genes and antioxidant enzyme genes was studied. The antioxidant potential of extracts were also evaluated *in tubo* with DPPH (2,2-diphenyl-1-picrylhydrazyle) reduction test and Fenton reactions with divalent cations. Finally, elastin production was qualitatively assessed by orcein coloration of elastin fibers, after incubation of cell culture with or without extract for 6 days.

### Results

### (i) Chemical characterization of the extracts

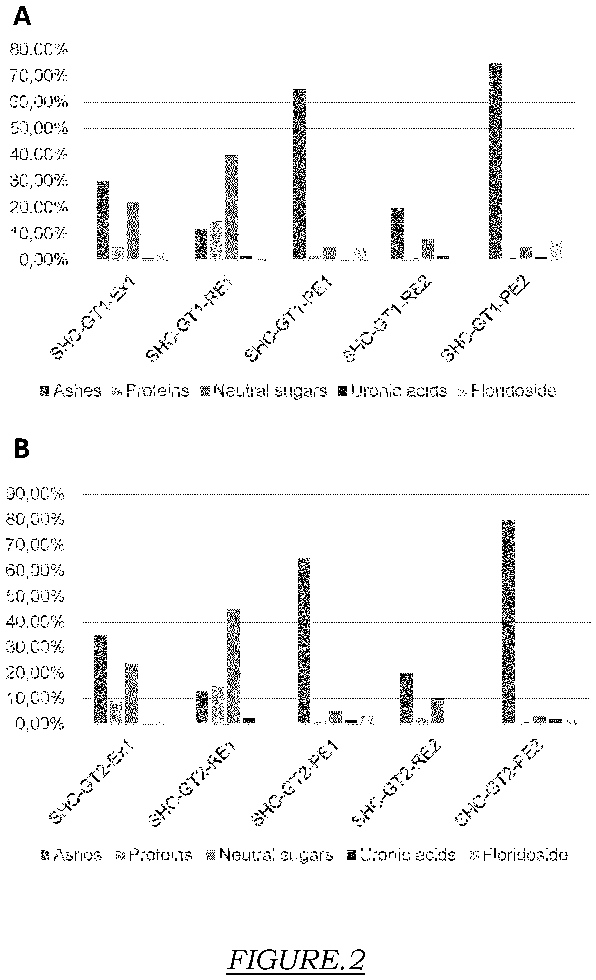

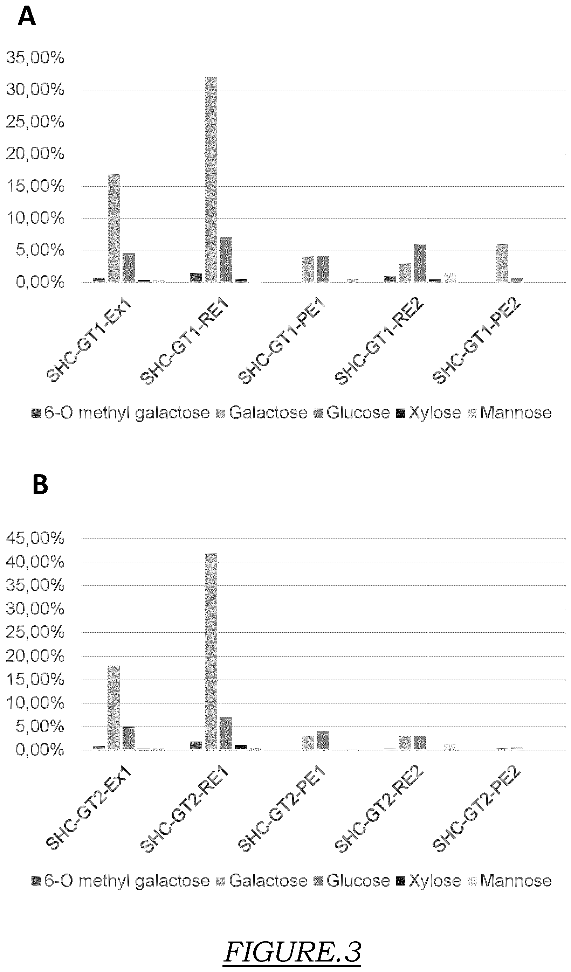

Ten extracts were obtained: five extracts from fresh biomass (GT1-XXX) and five extracts from dried biomass (GT2-XXX) (*see* *Figure 1*). The dry matter (DM) of *Grateloupia turuturu* extracts was analyzed (*see* *Figure 2* *and* *3*). GT1 and GT2 extracts were similar regarding their chemical composition. All extracts displayed non-significant amounts of phenol compounds and digeneasides (less than 0.01% of DM). The EX1, PE1 and PE2 extracts contained less floridosides than the other extracts. EX1 extracts were rich in proteins and ashes. The GT1-PE2 was rich in ashes (78 %) and floridosides (5%).

Regarding the sugar composition, the EX1 extract and RE1 extract were rich in polysaccharides. The GT2-EX1 extract contained more polysaccharides (40%) than the GT1-EX1 extracts (30%). The RE1 extract was rich in galactan polysaccharides (more than 40%). Glucose polysaccharides were present in EX1 (5-8%), RE1 (3-5%) and RE2 fractions (6-8%).

### (ii) In tubo activity screening of the 10 extracts

The extracts were assessed regarding their biological and chemical activities. According to the results (*see Table 1 below*), the *in tubo* biological activities were different between extracts obtained from fresh or dried biomass, despite their similar chemical composition.

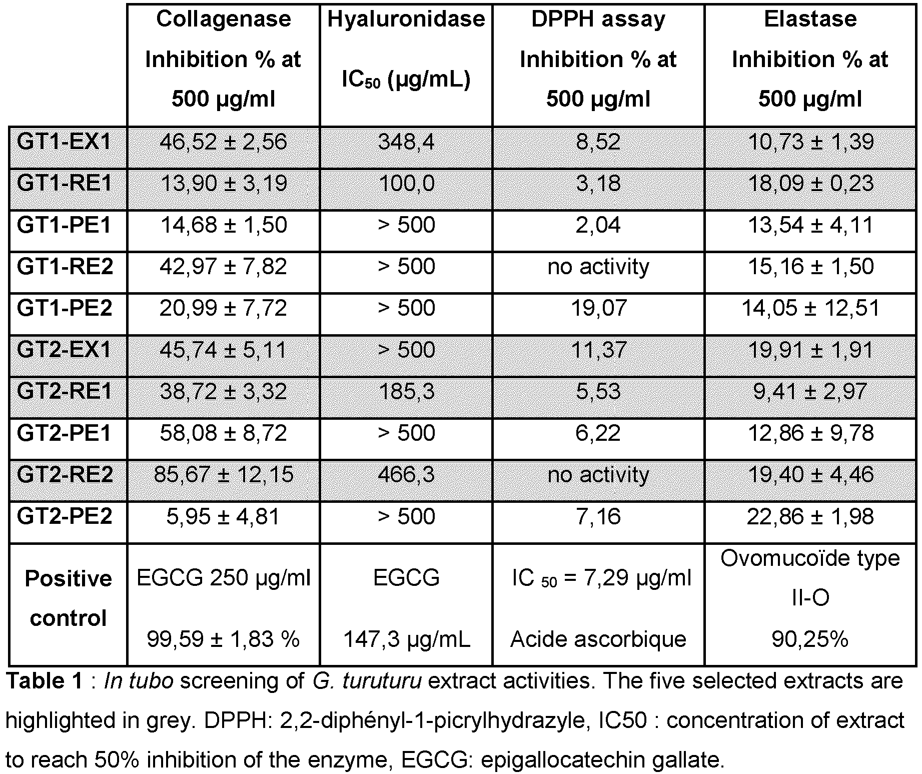

Regarding the inhibition of collagenase activity, GT2-RE2, GT2-PE1, GT1-EX1 were the more efficient extracts for inhibiting enzymatic degradation of collagen with 86%, 58% and 46% of inhibition, respectively, at 500 µg/mL. As regard to the hyaluronidase activity, GT1-RE1, GT2-RE1 and GT1-EX1 were the only extracts to display interesting inhibition activities with an IC50 of 100 µg/mL, 185 µg/mL and 348 µg/mL, respectively. Regarding the elastase inhibition test, GT2-PE2 (23%), GT2-EX1 (20%) and GT2-RE2 (19%) were the most efficient. For the DDPH inhibition test, the efficient extracts were GT1-PE2 (19%), GT2-EX1 (11 %) and GT1-EX1 (9%)

Extracts GT1-EX1, GT1-RE1, GT2-EX1, GT2-RE1, GT2-RE2 were selected for further assessments.

No irritation nor cytotoxicity was observed on VERO cells and in the HET-CAM test, respectively, for the 5 selected extracts.

### (iii) Selection of the active ingredient

The 5 extracts were then assessed for their *in vitro* biological activities on senescent fibroblasts and *in tubo* for their chemical antioxidant activities. The biological and chemical properties regarding their well-ageing are detailed thereafter. The GT1-RE1 was selected as active ingredient to perform well-ageing cosmetic product.

### Detailed activities of the five selected extracts

### ∘ GT1-RE1 biological and chemical activity

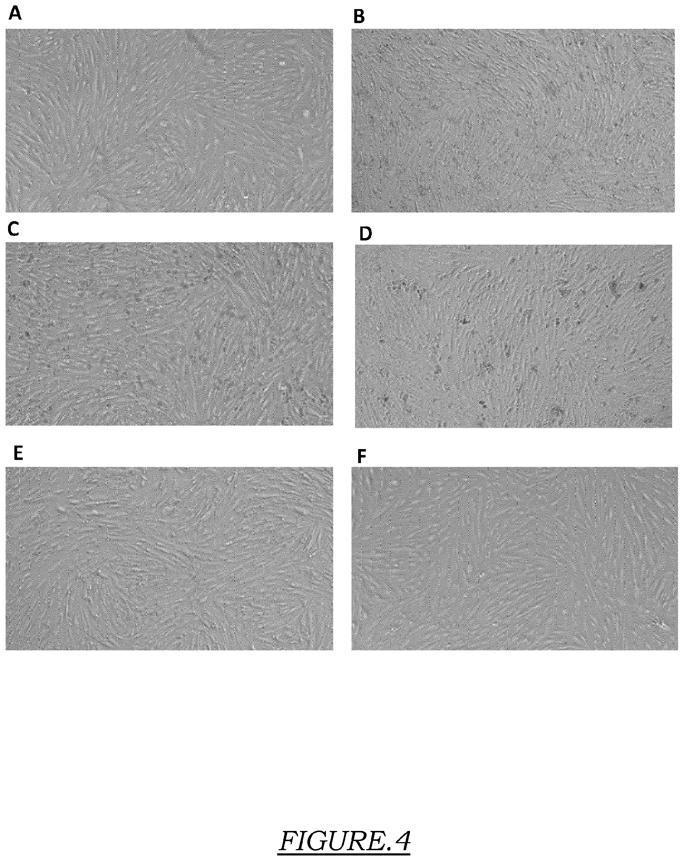

The GT1-RE1 extract were selected according to the *in tubo* and *in vitro* anti-aging activities. The GT1-RE1 did not induce any morphological change in senescent fibroblasts but induced cell proliferation (*see* *Figure 4*).

The GT1-RE1 extract was also able to reduce the oxidative stress. GT1-RE1 inhibited the Fenton reaction with Zn²⁺ ions and stimulated, in non-oxidative and oxidative H₂O₂ conditions, the expression of the superoxide dismutase gene (400 % and 497 %, respectively, at 625 µg/mL) and catalase gene (272 % and 308 %, respectively, at 625 µg/mL).

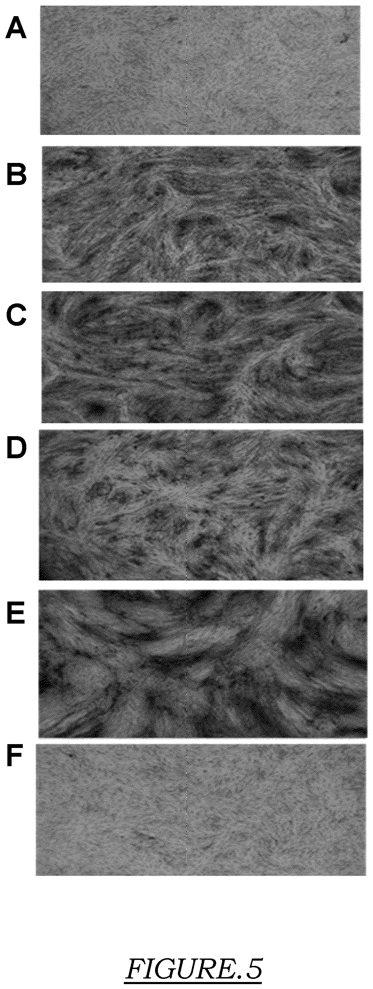

The GT1-RE1 extract reduced the synthesis and the activity of extracellular protease, degrading the dermis extracellular matrix. The GT1-RE1 extract reduced the expression of ADAMT5 (65 % at 625 µg/mL), MMP2 (87 % at 625 µg/ml) and MMP3 (65% at 625 µg/mL) genes from senescent fibroblasts. It also inhibited the activities of hyaluronidase (IC50 = 100 µg/mL), collagenase (14 % at 500 µg/mL) and elastase (18 % at 500 µg/mL). The coloration of elastic fibers with orcein showed that, in the presence of the GT1-RE1 extract, the elastin rate is higher, and that elastin fibers display a better structural organization, compared to the control without extract (*see* *Figure 5*)*.*

### ∘ GT1-EX1 biological and chemical activity

The GT1-EX1 extract did not induce any morphological change in senescent fibroblasts but induced cell proliferation (*see* *Figure 4*).

The GT1-EX1 extract decreased the expression of a senescence marker gene, the β-galactosidase (39% at 625 µg/mL).

The GT1-EX1 extract was also able to reduce the oxidative stress. GT1-EX1 inhibited the Fenton reaction with Cu²⁺ and Zn²⁺ ions and stimulated, in non-oxidative and oxidative H₂O₂ conditions, the expression of superoxide dismutase gene (850 % and 364 %, respectively, at 625 µg/mL) and catalase gene (240 % and 212 %, respectively at 625 µg/mL).

The GT1-EX1 extract reduces the synthesis and the activity of extracellular protease, degrading the dermis extracellular matrix. The GT1-RE1 extract reduced the expression on ADAMT5 (85 % at 625 µg/mL), MMP2 (93 % at 625 µg/ml) genes from senescent fibroblasts. It also inhibited the activities of collagenase (46 % at 500 µg/mL) and elastase (10 % at 500 µg/mL). The coloration of elastic fibers with orcein showed that, in the presence of the GT1-EX1 extract, the elastin rate was higher and that elastin fibers displayed a better structural organization, compared to the control without extract (*see* *Figure 5*)*.*

### ∘ GT2-RE1 biological and chemical activity

The GT2-RE1 extract did not induce any morphological change in senescent fibroblasts but induced cell proliferation (Figure 4).

The GT2-RE1 extract was also able to reduce the oxidative stress by inhibiting the Fenton reaction with Fe²⁺ ions.

The GT2-RE1 extract reduced the synthesis and the activity of extracellular protease, degrading the dermis extracellular matrix. The GT2-RE1 extract reduced the expression on ADAMT5 gene (30 % at 625 µg/mL) from senescent fibroblasts. It also inhibited the activities of collagenase (39 % at 500 µg/mL) and hyaluronidase (IC50 = 185 µg/mL). The coloration with orcein showed that, in the presence of the GT2-RE1 extract, the elastin rate was higher and that elastin fibers displayed a better structural organization, compared to the control without extract (*see* *Figure 5*).

### ∘ GT2-EX1 biological and chemical activities

The GT2-EX1 extract did not induce any morphological change in senescent fibroblasts (*see* *Figure 4*).

The GT2-RE1 extract was also able to reduce the oxidative stress by inhibiting the Fenton reaction with Zn²⁺ ions.

The GT2-RE1 extract reduced the synthesis and the activity of extracellular protease, degrading the dermis extracellular matrix. The GT2-RE1 extract reduced the expression on ADAMT5 gene (66 % at 625 µg/mL) from senescent fibroblasts. It also inhibited the matrix degrading enzymes collagenase (46 % at 500 µg/mL) and elastase (20% at 500 µg/mL).

### ∘ GT2-RE2 biological and chemical activity

The GT2-RE2 extract did not induce any morphological change in senescent fibroblasts (*see* *Figure 2*). The GT2-RE1 extract was able to reduce the oxidative stress by inhibiting the Fenton reaction with Cu²⁺ and Fe²⁺ ions.

The GT2-RE2 reduced the activity of extracellular protease, degrading the dermis extracellular matrix. The GT2-RE1 inhibited the matrix degrading enzymes collagenase (86 % at 500 µg/mL) and elastase (19% at 500 µg/mL).

## Claims

1. A red algae extract, wherein said red algae is *Grateloupia turuturu.*

2. The red algae extract according to claim 1, wherein said extract is obtained by a method comprising an aqueous hot extraction, a solid / liquid separation and a filtration.

3. The red algae extract according to claim 1 or 2, wherein sais extract has the following properties:
- inhibition of at least one enzyme selected from the group consisting of collagenase, elastase and hyaluronidase,
- optionally, reduction of the expression of at least one extracellular protease, said extracellular protease being selected from the group consisting of ADAMT5, MMP2 and MMP3,
- optionally, induction of cell proliferation of senescent fibroblasts, and
- optionally, decrease of oxidative stress.

4. A cosmetic composition comprising the red algae extract according to any one of claims 1 to 3.

5. A process for preparing a *Grateloupia turuturu* red algae extract, said process comprising:
a) providing *Grateloupia turuturu* red algae,
b) subjecting said red algae to an aqueous hot extraction, thereby obtaining a mixture,
c) subjecting said mixture to a solid / liquid separation, thereby obtaining a liquid fraction,
d) filtering said liquid fraction, thereby obtaining a clarified liquid fraction,
e) optionally, tangential ultrafiltering and diafiltering said clarified liquid fraction, thereby obtaining a first retentate and a first permeate, and
f) optionally, tangential ultrafiltering and diafiltering said first permeate, thereby obtaining a second retentate and a second permeate.

6. The process according to claim 5, wherein the algae provided in step a) are (i) fresh algae, (ii) fresh, freezed and defrosted algae or (iii) dried algae.

7. The process according to claim 5 or 6, wherein the aqueous hot extraction comprises macerating the red algae in water at a temperature comprised from 50°C to 80°C.

8. The process according to any one of claims 5 to 7, wherein the solid / liquid separation is a centrifugation or a decantation.

9. The process according to any one of claims 5 to 8, wherein tangential ultrafiltering and diafiltering in step e) is carried out with a filter having a pore size of 150 kDa and/or tangential ultrafiltering and diafiltering in step f) is carried out with a filter having a pore size of 3 Da.

10. A process for preparing a cosmetic composition, wherein said process comprises mixing the red algae extract according to any one of claims 1 to 3 or obtained by the process according to any one of claims 5 to 9 with at least one other cosmetic ingredient.

11. A cosmetic method comprising applying topically a cosmetic composition according to claim 4 or obtained by the process according to claim 10.

12. The cosmetic method according to claim 11, for slowing ageing of the skin.

13. Use of a red algae extract according to any one of claims 1 to 3 or obtained by the process according to any one of claims 5 to 9, as a cosmetic active ingredient.

14. The use of a red algae extract according to claim 13, as a slow-ageing agent.
